Europäisches Patentamt

European Patent Office

Office européen des brevets

(19)

(11)  **EP 0 805 801 B1**

(12)  **FASCICULE DE BREVET EUROPEEN**

(45) Date de publication et mention
de la délivrance du brevet:
**04.10.2001  Bulletin 2001/40**

(51) Int Cl.⁷: **C07D 201/08**

(21) Numéro de dépôt: **96901830.8**

(86) Numéro de dépôt international:
**PCT/FR96/00102**

(22) Date de dépôt: **22.01.1996**

(87) Numéro de publication internationale:
**WO 96/22974 (01.08.1996 Gazette 1996/35)**

(54) **PROCEDE DE PREPARATION DE LACTAME**

VERFAHREN ZUR HERSTELLUNG VON LACTAMEN

METHOD FOR PREPARING LACTAM

(84) Etats contractants désignés:
**BE DE FR GB IT NL**

(30) Priorité:  **27.01.1995  FR 9501183**

(43) Date de publication de la demande:
**12.11.1997  Bulletin 1997/46**

(73) Titulaire: **RHODIA POLYAMIDE INTERMEDIATES**
**69190 Saint-Fons (FR)**

(72) Inventeurs:
• **COTTING, Marie-Christine**
**F-69500 Bron (FR)**
• **GILBERT, Laurent**
**F-69007 Lyon (FR)**
• **LAURAIN, Nathalie**
**F-69003 Lyon (FR)**
• **NEDEZ, Christophe**
**F-92600 Asnières-sur-Seine (FR)**

(74) Mandataire: **Esson, Jean-Pierre et al**
**Rhodia Services,**
**Direction de la Propriété Industrielle,**
**Centre de Recherches de Lyon,**
**BP 62**
**69192 Saint-Fons Cédex (FR)**

(56) Documents cités:
**EP-A- 0 015 801**          **EP-A- 0 097 539**
**EP-A- 0 659 741**          **DE-A- 4 339 648**
**FR-A- 1 166 597**          **FR-A- 2 029 540**
**US-A- 2 357 484**          **US-A- 4 625 023**
**US-A- 4 628 085**

Remarques:
Le dossier contient des informations techniques
présentées postérieurement au dépôt de la
demande et ne figurant pas dans le présent
fascicule.

## Description

La présente invention concerne la préparation de lactame par hydrolyse cyclisante d'aminonitrile correspondant.

Les lactames aliphatiques, tels que notamment l'epsilon-caprolactame, sont des composés de base pour la préparation des polyamides (polyamide 6 à partir du caprolactame).

Un des moyens connus de préparer ces lactames consiste à effectuer une hydrolyse cyclisante des aminonitriles correspondants, plus particulièrement des aminonitriles aliphatiques non ramifiés, par passage en phase vapeur avec de l'eau sur un catalyseur solide.

Ainsi le brevet US 2 357 484 décrit un procédé de préparation en phase vapeur de lactame, consistant à faire passer un mélange d'eau et d'aminonitrile sur un catalyseur de déshydratation, tel qu'alumine activée, gel de silice ou acide borophosphorique.

Le brevet US 4 628 085 a proposé un procédé de préparation de lactames en phase vapeur, consistant à mettre en contact un aminonitrile aliphatique ou aromatique et de l'eau avec un catalyseur à base de silice, sous forme de particules sphériques ayant une surface BET supérieure à 250m$^2$/g et un diamètre moyen de pores inférieur à 20 nm, et généralement en présence d'hydrogène et d'ammoniac.

Les catalyseurs utilisés dans les procédés de l'art antérieur peuvent permettre le cas échéant d'obtenir de bonnes sélectivités en lactame. Par contre, leur désactivation peut également être rapide, ce qui constitue un très gros handicap pour une mise en oeuvre industrielle desdits procédés.

En outre, le procédé selon US 4 628 085 met en oeuvre un mélange réactionnel très complexe, nécessitant en fin de réaction des opérations de séparation et des recyclages qui compliquent beaucoup ledit procédé.

La présente invention propose de nouveaux catalyseurs alumines qui, tout en conduisant à une bonne sélectivité de la réaction de transformation des aminonitriles en lactames, ont une grande durée de vie et nécessitent donc une régénération moins fréquente.

Plus précisément, l'invention consiste en un procédé de préparation de lactame par réaction en phase vapeur d'un aminonitrile aliphatique de formule générale (I) :

$$N \equiv C\text{-}R\text{-}NH_2 \qquad\qquad (I)$$

dans laquelle R représente un radical alkylène ayant de 3 à 12 atomes de carbone, avec de l'eau, en présence d'un catalyseur solide, caractérisé en ce que le catalyseur est une alumine ayant une surface spécifique mesurée par la méthode BET supérieure ou égale à 10 m$^2$/g.

Parmi les aminonitriles de formule (I) les plus importants sont ceux qui conduisent aux lactames servant de matière première pour la préparation des polyamides 4, 5, 6 et 10, c'est-à-dire ceux dans la formule desquels le symbole R représente un radical alkylène linéaire ayant 3, 4, 5 ou 9 atomes de carbone.

Le composé de formule (I) préféré est l'amino-6 capronitrile (ou epsilon-capronitrile), qui conduit au caprolactame dont la polymérisation fournit le polyamide 6.

Les alumines qui peuvent être utilisées dans le présent procédé sont tout d'abord les alumines ayant une surface spécifique supérieure ou égale à 50 m$^2$/g et inférieure ou égale à 280 m$^2$/g ainsi qu'un volume des pores de diamètre supérieur à 70 angstroms supérieur ou égal à 30 ml/100 g.

Selon une autre caractéristique, les alumines présentant, en plus, un volume des pores de diamètre supérieur à 500 angstroms supérieur ou égal à 10 ml/100 g sont particulièrement convenables pour l'invention.

La surface spécifique BET est la surface spécifique déterminée par adsorption d'azote conformément à la norme ASTM D 3663-78 établie à partir de la méthode BRUNAUER-EMMETT-TELLER décrite dans le périodique "The Journal of the American Society", 60, 309 (1938).

Le volume des pores de diamètre supérieur à 500 Å représente le volume cumulé créé par tous les pores de taille supérieure à un diamètre de 500 Å. Ce volume est mesuré par la technique de la pénétration du mercure, dans laquelle on applique la loi de Kelvin.

De préférence, les alumines présentent un volume des pores de diamètre supérieur à 500 Å supérieur ou égal à 20 ml/100 g et encore plus préférentiellement supérieur ou égal à 30 ml/100 g.

De préférence, les alumines convenables pour l'invention présentent un volume des pores de diamètre supérieur à 70 Å supérieur ou égal à 45 ml/100 g.

De préférence également les alumines convenables pour l'invention présentent une surface spécifique supérieure ou égale à 80 m$^2$/g.

Les alumines sont aussi caractérisées par leur acidité.

Cette acidité peut être mesurée par le test d'isomérisation du butène-1 en butène-2.

Ce test est basé sur la réaction d'isomérisation du butène-1 en un mélange de cis-butène-2 et trans-butène-2 à une température T (T = 400°C dans le cas présent).

La réaction d'isomérisation est un équilibre thermodynamique. On peut définir deux constantes :

- la constante d'équilibre théorique Kth(T) déterminée par le calcul :

$$Kth(T) = \frac{[\text{cis-butène-2}]eq + [\text{trans-butène-2}]eq}{[\text{butène-1}]eq + [\text{cis-butène-2}]eq + [\text{trans-butène-2}]eq}$$

où [butène]eq représente la concentration de chacun des isomères en équilibre à la température T ;

- la constante d'équilibre réelle K(T) déterminée par le résultat des mesures :

$$K(T) = \frac{[\text{cis-butène-2}] + [\text{trans-butène-2}]}{[\text{butène-1}] + [\text{cis-butène-2}] + [\text{trans-butène-2}]}$$

où [butène] représente la concentration de chacun des isomères en sortie du réacteur à la température T.

Le pouvoir isomérisant A des alumines est défini par l'activité par rapport à l'équilibre

$$A(T) = \frac{K(T)}{Kth(T)} \times 100$$

En pratique le test est réalisé dans un réacteur phase vapeur fonctionnant en mode pulsé, dans lequel on introduit 500 mg d'alumine broyée (particules comprises entre 400 et 500 µm). L'alumine est conditionnée pendant 2 heures à 250°C sous courant d'hélium avec un débit de 2,5 litre/heure. Puis l'alumine est portée à une température de 400°C et on injecte en amont de celle-ci 1 millilitre de butène-1 dans le flux d'hélium. L'analyse des gaz de sortie est réalisée par chromatographie en phase gazeuse et permet de mesurer les quantités de butène-1 et de butène-2 cis et trans récupérées.

Ce pouvoir isomérisant A est corrigé du pouvoir isomérisant obtenu dans les mêmes conditions avec le réacteur vide. Le pouvoir isomérisant corrigé $A_c$ représente l'acidité desdites alumines

Lorsque la teneur en métal alcalin ou alcalino-terreux présent dans l'alumine est inférieure à 60 mmol par 100 g d'alumine, plus la valeur de $A_c$ est élevée, plus l'alumine est acide.

Généralement les alumines sont obtenues par déhydratation de gibbsite, de bayerite, de nordstrandite ou de leurs différents mélanges. On peut se référer par exemple à l'encyclopédie KIRK-OTHMER, volume 2, pages 291-297.

On peut préparer les alumines mises en oeuvre dans le présent procédé par mise en contact d'une alumine hydratée, sous forme finement divisée, avec un courant de gaz chaud à une température comprise entre 400°C et 1000°C, puis maintien du contact entre l'hydrate et les gaz pendant une durée allant d'une fraction de seconde jusqu'à 10 secondes et enfin séparation de l'alumine partiellement déshydratée et des gaz chauds. On peut notamment se référer au procédé décrit dans le brevet américain US 2 915 365.

On peut également procéder à l'autoclavage d'agglomérés des alumines obtenues précédemment, en milieu aqueux, éventuellement en présence d'acide, à une température supérieure à 100°C et de préférence comprise entre 150°C et 250°C, pendant une durée de préférence comprise entre 1 et 20 heures, puis à leur séchage et leur calcination.

La température de calcination est réglée de telle façon que l'on obtienne des surfaces spécifiques et des volumes poreux situés dans les zones de valeurs indiquées précédemment.

En raison de leurs principaux procédés de fabrication, les alumines mises en oeuvre dans le présent procédé contiennent le plus souvent du sodium, dont la teneur est habituellement exprimée en poids de $Na_2O$ par rapport au poids de l'alumine.

Le catalyseur peut être mis en oeuvre sous diverses formes telles que poudre, billes, concassés, extrudés, pastilles, la mise en forme pouvant éventuellement être réalisée à l'aide d'un liant.

Il peut s'agir tout d'abord de billes d'alumine issues d'une mise en forme par oil-drop (ou coagulation en gouttes). Ce type de billes peut par exemple être préparé par un procédé selon l'enseignement des brevets EP-A-0 015 801 ou EP-A-0 097 539. Le contrôle de la porosité peut être réalisé en particulier, selon le procédé décrit dans le brevet EP-A-0 097 539, par coagulation en gouttes d'une suspension ou d'une dispersion aqueuse d'alumine ou d'une solution d'un sel basique d'aluminium se présentant sous la forme d'une émulsion constituée d'une phase organique, d'une phase aqueuse et d'un agent de surface ou d'un émulsionnant. Ladite phase organique peut en particulier être un hydrocarbure.

Il peut également s'agir de concassés d'alumine. Ces concassés peuvent être issus du concassage de tout type de matière à base d'alumine telle que, par exemple, des billes obtenues par tous types de procédé (oil-drop, drageoir ou tambour tournant) ou des extrudés. Le contrôle de la porosité de ces concassés se fait par le choix de la matière à base d'alumine que l'on concasse pour les obtenir.

Il peut aussi s'agir d'extrudés alumine. Ceux-ci peuvent être obtenus par malaxage, puis extrusion d'une matière à base d'alumine, ladite matière pouvant être issue de la déshydratation rapide d'hydrargillite ou de la précipitation d'un gel d'alumine. Le contrôle de la porosité de ces extrudés peut être réalisé par le choix de l'alumine mise en oeuvre et par les conditions de préparation de cette alumine ou par les conditions de malaxage de cette alumine avant extrusion. L'alumine peut ainsi être mélangée lors du malaxage à des porogènes. A titre d'exemple, les extrudés peuvent être préparés par le procédé décrit dans le brevet US 3 856 708.

Il peut dans certain cas être avantageux qu'au moins une partie du volume libre du réacteur soit occupé par un solide inerte, tel que par exemple du quartz, afin de favoriser la vaporisation et la dispersion des réactifs.

La réaction d'hydrolyse cyclisante nécessite la présence d'eau. Le rapport molaire entre l'eau et l'aminonitrile engagés se situe habituellement entre 0,5 et 50 et de préférence entre 1 et 20. La valeur supérieure de ce rapport n'est pas critique pour l'invention, mais des rapports plus élevés n'ont guère d'intérêt pour des questions économiques.

L'aminonitrile et l'eau peuvent être engagés sous forme de leurs mélanges à l'état de vapeurs ou être introduits séparément dans le réacteur. On peut réaliser une prévaporisation des réactifs qui circulent ensuite dans une chambre de mélange.

On peut sans inconvénient utiliser tout gaz inerte comme vecteur, tel que l'azote, l'hélium ou l'argon.

La température à laquelle est mis en oeuvre le procédé de l'invention doit être suffisante pour que les réactifs soient bien à l'état de vapeurs. Elle se situe généralement entre 200°C et 450°C et de préférence entre 250°C et 400°C.

Le temps de contact entre l'aminonitrile et le catalyseur n'est pas critique. Il peut varier selon l'appareillage utilisé notamment. Ce temps de contact se situe de préférence entre 0,5 à 200 secondes et encore plus préférentiellement entre 1 et 100 secondes.

La pression n'est pas un paramètre critique du procédé. Ainsi on peut opérer sous des pressions de $10^{-3}$ bar à 200 bar. De préférence, on mettra en oeuvre le procédé sous une pression de 0,1 à 20 bar.

Il n'est pas exclu d'utiliser un solvant inerte dans les conditions réactionnelles, tel que par exemple un alcane, un cycloalcane, un hydrocarbure aromatique ou l'un de ces hydrocarbures précédents halogéné, et d'avoir ainsi une phase liquide dans le flux réactionnel.

Les exemples qui suivent illustrent l'invention.

## EXEMPLES 1 A 4

Dans un réacteur cylindrique de 20 ml en verre Pyrex, disposé verticalement et muni de moyens de chauffage, d'ouvertures pour l'arrivée et la sortie des flux gazeux et d'un système d'injection des réactifs, on charge successivement 10 ml de quartz, 1ml du catalyseur sous forme de poudre de 0,8 à 1,25 micromètre (nature du catalyseur indiquée dans le tableau 1 ci-après) et à nouveau 10 ml de quartz.

Le réacteur ainsi chargé est chauffé à 400°C sous courant d'air (avec un débit de 1,5 litre/heure) pendant 2 heures. Ensuite le réacteur est refroidi à 320°C ( température de réaction choisie) et mis sous courant d'azote (débit de 1 litre/heure).

On injecte alors, à l'aide d'une pompe, un mélange d'amino-6 capronitrile (ACN) et d'eau (rapport pondéral 50/50, soit un rapport molaire eau/ACN de 6,2). La vitesse d'injection du mélange est de 1,2 ml/h.

A la sortie du réacteur, les vapeurs sont condensées dans un piège en verre à température ambiante, sur une durée de 2 heures.

Le mélange réactionnel final est dosé en chromatographie en phase vapeur.

On détermine le taux de transformation (TT) de l'aminocapronitrile, le rendement (RT) en caprolactame (CPL) par rapport à l'aminocapronitrile transformé et l'activité du catalyseur sur 2 heures de réaction, mesurée en grammes de caprolactame formé par millilitre de lit catalytique et par heure.

Les alumines utilisées comme catalyseurs présentent les caractéristiques suivantes :

- Alumine 7 :

  - acidité $A_c$ (400°C) = 62 %
  - surface spécifique (SS) = 81 m$^2$/g
  - 0,0714 % de $Na_2O$
  - volume des pores de diamètre supérieur à 500 Å : 27 ml/100 g.

- Alumine 6 :

  - acidité $A_c$ (400°C) = 65 %

- SS = 244 m$^2$/g
- 0,0730 % de Na$_2$O
- volume des pores de diamètre supérieur à 500 Å : 12 ml/100 g.

- Alumine 16 :

  - acidité A$_c$ (400°C) = 65 %
  - SS = 314 m$^2$/g
  - 0,3640 % de Na$_2$O
  - volume poreux total : 40 ml/100 g

- Alumine 10 : - acidité A$_c$ (400°C) = 99 %

  - SS = 217 m$^2$/g
  - 0,0030 % de Na$_2$O
  - volume des pores de diamètre supérieur à 70 Å : 45 ml/100 g.

Le tableau 1 ci-après rassemble les résultats obtenus.

Tableau 1

| Exemples | Catalyseur | TT % ACN | RT % CPL | Activité |
|---|---|---|---|---|
| Exemple 1 | alumine 7 | 84,2 | 88,3 | 0,46 |
| essai comparatif | alumine 6 | 96,0 | 90,0 | 0,53 |
| essai comparatif | alumine 16 | 96,0 | 88,0 | 0,66 |
| Exemple 4 | alumine 10 | 92,6 | 95,8 | 0,68 |

### EXEMPLES 5 A 7

On répète l'exemple 1 et les essais comparatifs, en suivant l'évolution de l'activité des différents catalyseurs sur des durées allant jusqu'à 32 heures.
Le tableau 2 ci-après rassemble les valeurs de l'activité pour chaque catalyseur et pour des durées croissantes de réaction.
On peut observer que les alumines mises en oeuvre ne perdent pas leur activité catalytique sur une durée d'au moins 32 heures.

Tableau 2

| Exemples | Catalyseur Alumine | Activité du catalyseur | | | | pour des durées de | | |
|---|---|---|---|---|---|---|---|---|
| | | 4 h | 6 h | 8 h | 10 h | 25 h | 30 h | 32 h |
| Exemple 5 | Alumine 7 | 0,45 | 0,49 | 0,47 | 0,45 | 0,52 | 0,48 | 0,47 |
| essai comparatif | Alumine 6 | 0,47 | 0,54 | 0,58 | 0,59 | 0,73 | 0,73 | 0,72 |
| essai comparatif | Alumine 16 | 0,75 | 0,77 | 0,77 | 0,78 | 0,75 | 0,75 | 0,75 |

### EXEMPLES 8 A 18 ET ESSAI COMPARATIF

Dans un réacteur cylindrique de 20 ml en verre Pyrex, disposé verticalement et muni de moyens de chauffage, d'ouvertures pour l'arrivée et la sortie des flux gazeux et d'un système d'injection des réactifs, on charge successivement 2 ml de quartz, 5 ml du catalyseur de granulométrie variant de 1 mm à 5 mm et à nouveau 5 ml de quartz.
Le réacteur ainsi chargé est chauffé à 350°C sous courant d'azote (avec un débit de 5,2 litres/heure) pendant 2 heures. Ensuite le réacteur est refroidi à 250°C (température de réaction choisie) et mis sous courant d'azote (débit de 5,2 litres/heure).
On injecte alors, à l'aide d'une pompe, un mélange d'amino-6 capronitrile (ACN) et d'eau (rapport molaire eau/ACN de 2,9). La vitesse d'injection du mélange liquide est de 14 g/h.
A la sortie du réacteur, les vapeurs sont condensées dans un piège en verre à température ambiante, sur une durée de 2 heures.

Le mélange réactionnel final est dosé en chromatographie en phase vapeur.

On détermine le taux de transformation (TT) de l'aminocapronitrile, le rendement (RT) en caprolactame (CPL) par rapport à l'aminocapronitrile transformé et l'activité du catalyseur sur 2 heures de réaction, mesurée en grammes de caprolactame formé par gramme de catalyseur et par heure (activité a) et en grammes de caprolactame formé par millilitre de lit catalytique et par heure (activité b).

Le taux de transformation de l'ACN varie de 25 % à 40 % dans les différents essais et le rendement RT en CPL est supérieur à 90 % pour les exemples 8 à 18 et est de 15 % pour l'essai comparatif avec l'alumine 15.

Les caractéristiques des alumines utilisées comme catalyseurs (surface spécifique = SS, volume poreux total = VPT, volume des pores de diamètre supérieur à 500 Å = V500 Å, volume des pores de diamètre supérieur à 70 Å = V70 Å) et les valeurs des activités a et b de ces différentes alumines sont indiquées dans le tableau 3 ci-après.

Tableau 3

| Exemples | Alumine | SS m$^2$/g | VPT ml/ 100g | V70Å ml/ 100g | V500Å ml/ 100g | A$_c$ % | Activité a | Activité b |
|---|---|---|---|---|---|---|---|---|
| Exemple 8 | alumine 1 | 139 | 117 | 116 | 50 | 93 | 2,06 | 0,79 |
| Exemple 9 | alumine 2 | 192 | 95 | 81 | 52 | 92 | 1,68 | 0,73 |
| Exemple 10 | alumine 3 | 190 | 72 | 65 | 31 | 92 | 1,32 | 0,66 |
| Exemple 11 | alumine 4 | 171 | 86 | 80 | 40 | 92 | 1,47 | 0,73 |
| essai comparatif | alumine 5 | 333 | 55 | 31 | 20 | 56 | 0,97 | 0,59 |
| essai comparatif | alumine 6 | 244 | 56 | 23 | 12 | 65 | 0,89 | 0,63 |
| Exemple 14 | alumine 7 | 81 | 68 | 66 | 27 | 62 | 0,86 | 0,44 |
| Exemple 15 | alumine 8 | 170 | 108 | 105 | 45 | 93 | 1,12 | 0,50 |
| Exemple 16 | alumine 9 | 115 | 72 | 69 | 29 | 70 | 1,09 | 0,45 |
| Exemple 17 | alumine 10 | 217 | 55 | 45 | 2 | 99 | 1,10 | 0,65 |
| Exemple 18 | alumine 11 | 191 | 60 | 58 | 1,5 | 98 | 0,98 | 0,59 |
| essai comparatif | alumine 12 | 352 | 43 | 17 | 8 | 84 | 0,91 | 0,59 |
| essai comparatif | alumine 13 | 408 | 37 | 14 | 7 | 100 | 0,84 | 0,68 |
| essai comparatif | alumine 14 | 350 | 43 | 15 | 6 | 28 | 0,87 | 0,65 |
| Essai comparatif | alumine 15 | 7,5 | 52 | 52 | 52 | 8 | 0,19 | 0,13 |

## EXEMPLES 22 A 27

Dans le réacteur décrit pour les exemples précédents, on charge successivement 3 ml de quartz, 2 ml du catalyseur de granulométrie variant de 1 mm à 5 mm et à nouveau 5 ml de quartz.

Le réacteur ainsi chargé est chauffé à 350°C sous courant d'azote (avec un débit de 5,2 litres/heure) pendant 2 heures. Ensuite le réacteur est maintenu à 350°C (température de réaction choisie) et mis sous courant d'azote (débit de 5,2 litres/heure).

On injecte alors, à l'aide d'une pompe, un mélange d'amino-6 capronitrile (ACN) et d'eau (rapport molaire eau/ACN de 1,1). La vitesse d'injection du mélange liquide est de 11 g/h.

A la sortie du réacteur, les vapeurs sont condensées dans un piège en verre à température ambiante, sur une durée indiquée dans le tableau 4 ci-après.

Le mélange réactionnel final est dosé en chromatographie en phase vapeur.

On détermine le taux de transformation (TT) de l'aminocapronitrile, le rendement (RT) en caprolactame (CPL) par rapport à l'aminocapronitrile transformé et l'activité du catalyseur sur la durée de réaction, mesurée en grammes de caprolactame formé par gramme de catalyseur et par heure (activité a) et en grammes de caprolactame formé par millilitre de lit catalytique et par heure (activité b).

Le taux de transformation de l'ACN est indiqué dans le tableau 4 et le rendement RT en CPL est supérieur à 90 % pour les exemples 22 à 27.

Les valeurs des activités a et b de ces différentes alumines sont indiquées dans le tableau 4.

Tableau 4

| Exemples | Alumine | Activité a | Activité b | TT % ACN | Durée du test |
|---|---|---|---|---|---|
| Exemple 22 | Alumine 1 | 8,1 | 3,0 | 66 | 52 h |
| Exemple 23 | Alumine 2 | 7,0 | 2,9 | 65 | 52 h |
| Exemple 24 | Alumine 4 | 5,4 | 2,7 | 59 | 49 h |
| essai comparatif | Alumine 6 | 4,5 | 2,6 | 56 | 48 h |
| Exemple 26 | Alumine 7 | 3,3 | 2,0 | 44 | 69 h |
| Exemple 27 | Alumine 10 | 4,7 | 2,7 | 59 | 48 h |
| essai comparatif | Alumine 12 | 3,6 | 2,6 | 54 | 48 h |

**Revendications**

1. Procédé de préparation de lactame par réaction en phase vapeur d'un aminonitrile aliphatique de formule générale (I):

$$N\equiv C\text{-}R\text{-}NH_2 \qquad\qquad (I)$$

dans laquelle R représente un radical alkylène ayant de 3 à 12 atomes de carbone, avec de l'eau, en présence d'un catalyseur solide, **caractérisé en ce que** le catalyseur est une alumine ayant une surface spécifique supérieure ou égale à 50 m²/g et inférieure ou égale à 280 m²/g ainsi qu'un volume des pores de diamètre supérieur à 70 angstroms supérieur ou égal à 30 ml/100 g.

2. Procédé selon la revendication 1, **caractérisé en ce que** l'alumine utilisée est choisie parmi les alumines présentant un volume des pores de diamètre supérieur à 500 Å supérieur ou égal à 10 ml/100 g

3. Procédé selon l'une des revendications 1 ou 2, **caractérisé en ce que** l'alumine utilisée est choisie parmi les alumines présentant un volume des pores de diamètre supérieur à 500 Å supérieur ou égal à 20 ml/100g.

4. Procédé selon l'une des revendications précédentes, **caractérisé en ce que** l'alumine utilisée est choisie parmi les alumines présentant un volume des pores de diamètre supérieur à 70 Å supérieur ou égal à 45 ml/100 g.

5. Procédé selon l'une des revendications 1 ou 4, **caractérisé en ce que** l'alumine utilisée est choisie parmi les alumines présentant une surface spécifique supérieure ou égale à 80 m²/g.

6. Procédé selon l'une des revendications 1 à 5, **caractérisé en ce que** l'aminonitrile de formule (I) est l'amino-6 capronitrile.

7. Procédé selon l'une des revendications 1 à 6, **caractérisé en ce que** le rapport molaire entre l'eau et l'aminonitrile engagés se situe entre 0,5 et 50 et de préférence entre 1 et 20.

8. Procédé selon l'une des revendications 1 à 7, **caractérisé en ce que** la température à laquelle il est mis en oeuvre se situe entre 200°C et 450°C et de préférence entre 250°C et 400°C.

**Patentansprüche**

1. Verfahren zur Herstellung von Lactam durch Umsetzung in einer Dampfphase von einem aliphatischen Aminonitril der allgemeinen Formel (I):

$$N\equiv C\text{-}R\text{-}NH_2 \tag{I}$$

worin R einen Alkylenrest mit 3 bis 12 Kohlenstoffatomen bedeutet, mit Wasser in Anwesenheit eines festen Katalysators, **dadurch gekennzeichnet, daß** der Katalysator ein Aluminiumoxid mit einer spezifischen Oberfläche von größer als oder gleich 50 $m^2$/g und geringer als oder gleich 280 $m^2$/g, sowie einem Volumen an Poren mit einem Durchmesser oberhalb 70 Angström von mehr als oder gleich 30 ml/100 g, ist.

2. Verfahren gemäß Anspruch 1, **dadurch gekennzeichnet, daß** das verwendete Aluminiumoxid unter den Aluminiumoxiden mit einem Volumen an Poren mit einem Durchmesser oberhalb 500 Angström von größer als oder gleich 10 ml/100g ausgewählt wird.

3. Verfahren gemäß einem der Ansprüche 1 oder 2, **dadurch gekennzeichnet, daß** das verwendete Aluminiumoxid unter den Aluminiumoxiden mit einem Volumen an Poren mit einem Durchmesser von oberhalb 500 Angström von mehr als oder gleich 20 ml/100 g ausgewählt wird.

4. Verfahren gemäß einem der vorhergehenden Ansprüche, **dadurch gekennzeichnet, daß** das verwendete Aluminiumoxid unter den Aluminiumoxiden mit einem Volumen an Poren mit einem Durchmesser von oberhalb 70 Angström von mehr als oder gleich 45 ml/100 g ausgewählt wird.

5. Verfahren gemäß einem der Ansprüche 1 bis 4, **dadurch gekennzeichnet, daß** das verwendete Aluminiumoxid unter den Aluminiumoxiden ausgewählt wird, die eine spezifische Oberfläche von mehr als oder gleich 80 $m^2$/g aufweisen.

6. Verfahren gemäß einem der Ansprüche 1 bis 5, **dadurch gekennzeichnet, daß** das Aminonitril der Formel (I) 6-Aminocapronitril ist.

7. Verfahren gemäß einem der Ansprüche 1 bis 6, **dadurch gekennzeichnet, daß** das Molverhältnis zwischen eingesetztem Wasser und Aminonitril zwischen 0,5 und 50 und vorzugsweise zwischen 1 und 20 liegt.

8. Verfahren gemäß einem der Ansprüche 1 bis 7, **dadurch gekennzeichnet, daß** die Temperatur, bei der es durchgeführt wird, zwischen 200°C und 450°C, und vorzugsweise zwischen 250°C und 400°C, liegt.

**Claims**

1. Process for the preparation of a lactam by vapour phase reaction of an aliphatic aminonitrile of general formula (I):

$$N\equiv C\text{-}R\text{-}NH_2 \tag{I}$$

in which R represents an alkylene radical having from 3 to 12 carbon atoms, with water in the presence of a solid catalyst, **characterized in that** the catalyst is an alumina having a specific surface greater than or equal to 50 $m^2$/g and less than or equal to 280 $m^2$/g and a volume for the pores with a diameter greater than 70 angstroms which is greater than or equal to 30 ml/100 g.

2. Process according to claim 1, **characterized in that** the alumina used is chosen from aluminas having a volume for the pores with a diameter greater than 500 Å which is greater than or equal to 10 ml/100 g.

3. Process according to either of claims 1 and 2, **characterized in that** the alumina used is chosen from aluminas exhibiting a volume for the pores with a diameter greater than 500 Å which is greater than or equal to 20 ml/100 g.

4. Process according to one of the preceding claims, **characterized in that** the alumina used is chosen from aluminas exhibiting a volume for the pores with a diameter greater than 70 Å which is greater than or equal to 45 ml/100 g.

5. Process according to either claim 1 or 4, **characterized in that** the alumina used is chosen from aluminas exhibiting a specific surface greater than or equal to 80 $m^2/g$.

6. Process according to one of claims 1 to 5, **characterized in that** the aminonitrile of formula (I) is 6-aminocapronitrile.

7. Process according to one of claims 1 to 6, **characterized in that** the molar ratio of water to aminonitrile charged is between 0.5 and 50 and preferably between 1 and 20.

8. Process according to one of claims 1 to 7, **characterized in that** the temperature at which it is implemented is between 200°C and 450°C and preferably between 250°C and 400°C.